# EUROPEAN PATENT SPECIFICATION

(11) **EP 3 457 952 B1**
(45) Date of publication and mention of the grant of the patent: **30.12.2020**
(21) Application number: 17781236.9
(22) Date of filing: 19.09.2017
(51) Int. Cl.: A61B 1/00, A61B 17/11, A61M 27/00, A61F 2/04

(54) **ANASTOMOTIC DRAINAGE STENT**
ANASTOMOTISCHER DRAINAGESTENT
ENDOPROTHÈSE DE DRAINAGE ANASTOMOTIQUE

(30) Priority: 19.09.2016 US 201662396524 P
(43) Date of publication of application: 27.03.2019
(73) Proprietor: Boston Scientific Scimed, Inc., Maple Grove, MN 55311-1566 (US)
(72) Inventor: GRAY, Jeff, Sudbury, MA 01776 (US); BANNON, Bryan, Duxbury, MA 02332 (US); DONOVAN, Ryan R., Santa Clara, CA 95051 (US); ECKERLINE, Katharine, Boston, MA 02113 (US)
(74) Representative: Vossius & Partner Patentanwälte Rechtsanwälte mbB
(86) International application number: PCT/US2017/052200
(87) International publication number: WO 2018/053477

(56) References cited:
- EP-A1- 0 716 834
- WO-A1-2012/007047
- WO-A2-2007/140079
- US-A1- 2004 193 093
- US-A1- 2004 193 093
- US-A1- 2005 177 144
- US-A1- 2009 143 713

## Description

### FIELD

The present disclosure relates to the field of body lumen drainage. Specifically, the present disclosure relates to implantable medical devices for facilitating the flow of fluids and materials between adjacent body lumens. In particular, the present disclosure relates to a drainage stent which maintains an open flow passage between opposed tissue layers.

### BACKGROUND

Gallstones and blockages of bile flow from the gallbladder to the common bile duct require approximately 800,000 gallbladder removal surgeries *(i.e.,* cholecystectomy) per year in the United States alone. Currently available drainage stents may not be indicated for permanent implantation within the patient, and relief from chronic gallstones and/or blockage of bile flow will ultimately require surgical intervention.

WO 2012/007047 A1 relates to a device for diverting bile from a gall bladder to a portion of an intestine. The device comprises a first portion adapted to be attached to a first lumen of the biliary tree and gallbladder, a second portion adapted to be attached to second lumen of a portion of intestine, a bile conduit portion connected with at least one of the first and second portion and adapted to define a flow path between the first lumen and the second lumen, a unidirectional valve arranged in the flow path of the bile conduit portion and adapted to prevent a flow from the second lumen to the first lumen.

### SUMMARY

The invention is defined in independent claim 1. Embodiments of the invention are recited in the dependent claims. In one aspect, the present disclosure provides a drainage conduit, comprising an elongate tubular body defining a first lumen, a proximal retention structure, a distal retention structure, and a cylindrical saddle region extending therebetween. A funnel member may be attached to a distal end of the elongate tubular body, wherein the funnel member may be configured to move between a first configuration and a second configuration, and wherein, when in the first configuration, the funnel member and elongate tubular body may define an open central lumen to provide a flow path therethrough. The funnel member may fold back along an outer surface of the elongate tubular body when in the second configuration. The funnel member may comprise a compliant or semi-compliant material. The proximal and distal retention structures may extend outward from an outer surface of the elongate tubular body. In addition, or alternatively, the proximal and distal retention structures may extend perpendicular to a circumference of the elongate tubular body. A diameter of the proximal and distal retention structures may be larger than a diameter of the cylindrical saddle region. The proximal and distal retention structures may include opposing planar surfaces configured to contact a surface of opposing tissue walls. The proximal retention structure may be configured to contact a first tissue layer, and the distal retention structure may be configured to contact a second tissue layer. A barrier member may be disposed about portion of the elongate tubular body extending proximally beyond the proximal retention structure. The barrier member may include a mesh-like structure. The drainage conduit may further include a valve disposed within the lumen of the elongate tubular body. The drainage conduit may further comprise a tab extending from an inner surface of the elongate tubular body into the first lumen. The tab may include an aperture therein. The tab may be disposed at a distal end of the elongate tubular body.

In another aspect, the present disclosure provides a drainage system, comprising a tissue anchor defining a first lumen, a proximal retention structure, a distal retention structure, and a cylindrical saddle region extending therebetween, and a funnel member defining a second lumen, wherein the funnel member may include a proximal flange configured to form an interference fit with an inner surface of the distal retention structure, and wherein the tissue anchor and funnel member may define an open central lumen when the proximal flange is disposed within the distal retention structure, thereby providing a flow path therethrough. The funnel member may comprise a compliant or semi-compliant material. The proximal flange may extend perpendicular to a proximal end of the funnel member. A diameter of the proximal flange may be larger than a diameter of the cylindrical saddle region. A valve may be disposed within the second lumen of the funnel member. The tissue anchor may include a self-expanding stent, including, for example a metallic stent.

In yet another aspect, the present disclosure provides a delivery system, comprising an endoscope with a proximal end, a distal end, and a lumen extending therebetween, and a drainage conduit disposed within the lumen of the endoscope. The drainage conduit may include an elongate tubular body defining a first lumen, a proximal retention structure, a distal retention structure, and a cylindrical saddle region extending therebetween. A funnel member may be attached to a distal end of the elongate tubular body, wherein the funnel member may be configured to move between a first configuration and a second configuration, and wherein, when in the first configuration, the funnel member and elongate tubular body may define an open central lumen to provide a flow path therethrough. A tab may extend from an inner surface of the elongate tubular body into the first lumen. The distal end of the endoscope may be disposed within the first lumen of the elongate tubular body and may contact the tab. A tether forming a loop may extend the length of the endoscope lumen and back along an outer surface of the endoscope, wherein the tether may be configured to maintain the funnel member in the second configuration.

### BRIEF DESCRIPTION OF THE DRAWINGS

Non-limiting examples of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment of the disclosure shown where illustration is not necessary to allow those of skill in the art to understand the disclosure. In the figures:
FIGS. 1A-1B illustrate side views of a drainage conduit in the deployed (FIG. 1A) and delivery (FIG. 1B) configurations, according to an embodiment of the present disclosure.
FIG. 2 illustrates a side view of a drainage conduit that includes a barrier member, according to an embodiment of the present disclosure.
FIG. 3 illustrates a side view of a drainage conduit that includes a barrier member, according to another embodiment of the present disclosure.
FIGS. 4A-4B illustrate side views of a drainage conduit that includes a one-way duck-bill valve in the closed (FIG. 4A) and open (FIG. 4B) configurations, according to an embodiment of the present disclosure.
FIGS. 5A-5B illustrate side views of a drainage conduit that includes a one-way pressure slit-valve in the closed (FIG. 5A) and open (FIG. 5B) configuration, according to another embodiment of the present disclosure.
FIGS. 6A-6B illustrate schematic views of a drainage conduit disposed within an anastomosis between the gallbladder and duodenum, according to an embodiment of the present disclosure.
FIGS. 7A-7C illustrate a method for replacing a temporary drainage stent with a drainage conduit, according to an embodiment of the present disclosure.
FIG. 8 illustrates a perspective view of a drainage conduit disposed on the distal end of an endoscope in a delivery configuration, according to an embodiment of the present disclosure.
FIGS. 9A-9C illustrate a drainage system, according to another embodiment of the present disclosure.
FIG. 10 illustrates the drainage system of FIG. 9C disposed within an anastomosis between the gallbladder and duodenum, according to an embodiment of the present disclosure.

It is noted that the drawings are intended to depict only typical or exemplary embodiments of the disclosure. Accordingly, the drawings should not be considered as limiting the scope of the disclosure. The disclosure will now be described in greater detail with reference to the accompanying drawings.

### DETAILED DESCRIPTION

Before the present disclosure is described in further detail, it is to be understood that the disclosure is not limited to the particular embodiments described, as such may vary. It is also to be understood that the terminology used herein is for the purpose of describing particular embodiments only, and is not intended to be limiting beyond the scope of the appended claims. Unless defined otherwise, all technical terms used herein have the same meaning as commonly understood by one of ordinary skill in the art to which the disclosure belongs. Finally, although embodiments of the present disclosure are described with specific reference to medical devices and systems for permanent drainage of the gallbladder, it should be appreciated that such medical devices may be used to establish and/or maintain a temporary or permanent anastomosis between a variety of body organs, lumens and spaces, *e.g*., the stomach and duodenum. Moreover, such medical devices are not limited to drainage, but may facilitate access to organs for other purposes, such as delivering therapy, including non-invasive manipulation of the tissue within the organ and/or the introduction of pharmacological agents via the anastomosis.

As used herein, the term "distal" refers to the end farthest away from a medical professional when introducing a device into a patient, while the term "proximal" refers to the end closest to the medical professional when introducing a device into a patient.

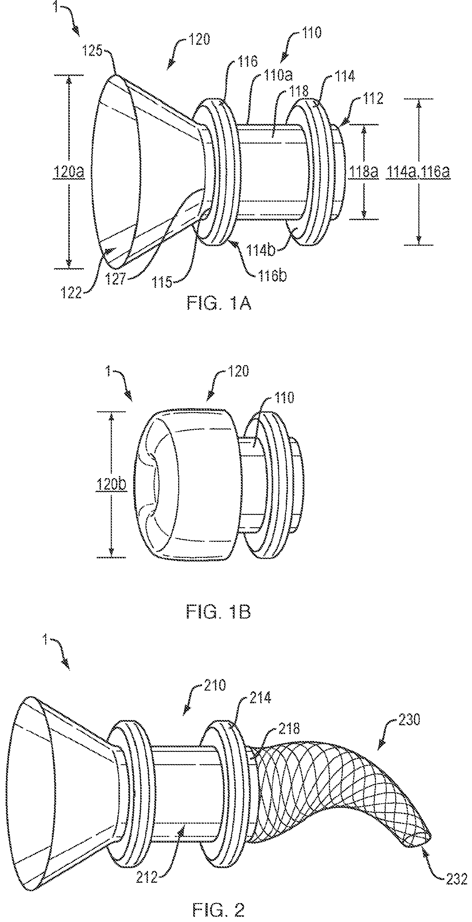

In one embodiment, the present disclosure provides a drainage conduit configured to fit within a previously formed anastomosis between opposed tissue layers. As illustrated in FIG. 1A, the drainage conduit 1 may include an elongate tubular body 110 defining a first lumen 112. The elongate tubular body 110 may include a cylindrical saddle region 118 with proximal and distal retention structures 114, 116 extending outward from an outer surface 110a of the elongate tubular body. The elongate tubular body 110 and proximal and distal retention structures 114, 116 may comprise a variety of non-degradable and biocompatible polymeric materials (e.g., upon exposure to bodily fluids such as bile), including, for example, silicones, rubbers, polyethylenes and thermoplastic elastomers. In one embodiment, the proximal and distal retention structures 114, 116 may be unitarily formed with the elongate tubular body 110 by polymeric extrusion process or injection molding process. In addition, or alternatively, the proximal and distal retention structures 114, 116 may be permanently affixed (*i.e*., adhered, bonded, attached etc.) to the outer surface 110a of the elongate tubular body 110 using suitable glues, adhesives, resins or other polymer bonding techniques.

A funnel member 120 may be attached to and extend distally beyond a distal end 115 of the elongate tubular body 110. The funnel member 120 may comprise a sufficiently pliable and/or deformable material to allow the funnel member to move between a first (*e.g*., deployed) configuration as depicted in FIG. 1A, and a lower-profile second (*e.g*., delivery) configuration as depicted in FIG. 1B. In one embodiment, the funnel member 120 and elongate tubular body 110 may be formed from the same biocompatible polymeric materials. Alternatively, the funnel member 120 and elongate tubular body may be formed from different biocompatible polymeric materials. For example, the funnel member 120 may comprise a polymeric material that is more compliant that the relatively less compliant polymeric materials of the elongate tubular body 110 and proximal and distal retention structures 114, 116. By way of non-limiting example, the funnel member 120 may include one or more thermoplastics and/or thermosets. Examples of thermoplastics include polyolefins; polyamides (*e.g*., nylon, such as nylon 12, nylon 11, nylon 6/12, nylon 6, nylon 66); polyesters (*e.g*., polyethylene terephthalate (PET), polybutylene terephthalate (PBT), polyethylene naphthalate (PEN), polytrimethylene terephthalate (PTT)); polyethers; polyurethanes; polyvinyls; polyacrylics; copolymers and block copolymers thereof, such as block copolymers of polyether and polyamide (*e.g*., PEBAX®); and mixtures thereof. Examples of thermosets include elastomers (*e.g*., EPDM), epichlorohydrin, polyureas, nitrile butadiene elastomers and silicones. Biocompatible thermosets may also be used. Biocompatible thermosets include, for example, biodegradable polycaprolactone, poly(dimethylsiloxane) containing polyurethanes and ureas and polysiloxanes.

When in the second configuration (FIG. 1B), the funnel member 120 may fold or roll back to lay against the distal retention structure (not visible) and a portion of the outer surface (not visible) of the elongate tubular body 110. When in the first configuration (FIG. 1A), the funnel member 120 may define a second lumen 122 which is coextensive with the first lumen 112 to form a contiguous open central lumen extending through the drainage conduit 1. In one embodiment, the funnel member 120 may be unitarily formed along with the elongate tubular body 110 by polymeric extrusion process, as is known in the art. In addition, or alternatively, a proximal end 127 of the funnel member may be permanently affixed (*i.e*., adhered, bonded, attached etc.) to the distal end 115 of the elongate tubular body 110 using suitable glues, adhesives, resins or other polymer bonding techniques, as discussed above. In one embodiment, the funnel member 120 may include a wall thickness that is substantially the same as a wall thickness of the elongate tubular body 110. Alternatively, the funnel member 120 may include a wall thickness that is less than the wall thickness of the elongate tubular body 110. For example, the elongate tubular body 110 may include a wall thickness of approximately 0.5-2.0 mm, and the funnel member 120 may include a wall thickness of approximately 0.1-0.5 mm. In one embodiment, the funnel member 120 may include a variable wall thickness which becomes progressively thinner from the proximal end 127 to the distal end 125 (*i.e*., as the funnel member increases in diameter).

Referring to FIG. 1A, the proximal and distal retention structures 114, 116 may extend perpendicularly from the outer surface about a circumference (*i.e*., 360°) of the outer surface 110a of elongate tubular body 110. The proximal and distal retention structures 114, 116 may have an outer diameter 114a, 116a that exceeds the outer diameter 118a of the cylindrical saddle region 118, but which is less than the outer diameter 120a of the funnel member 120 when in the first configuration. For example, the proximal and distal retention structures 114, 116 may have an outer diameter 114a, 116a of approximately 12.0-20.0 mm. The cylindrical saddle region 118 may have an outer diameter 118a of approximately 8.0-12.0 mm diameter, an inner diameter (*e.g*., first lumen 112) of approximately 6.0-10.0 mm, and a length of approximately 10.0-20.0 mm. The funnel member 120 may have an outer diameter 120a at the distal end 125 of approximately 20.0-50.0 mm when in the first configuration, and an outer diameter 120b approximately equal to the outer diameter 114a, 116a of the proximal and distal retention structures 114, 116 (*e.g*., approximately 12.0-20.0 mm) when in the second configuration (FIG. 1B). The proximal and distal retention structures 114, 116 may further include opposing planar surfaces 114b, 116b configured to contact the inner surfaces of opposing tissue layers (discussed below). If desired for a particular application, the retention structures may include a diameter that equals or exceeds the diameter of the funnel when deployed.

Referring to FIG. 2, the drainage conduit 1 may further include a barrier member 230 disposed about a portion of the cylindrical saddle region 218 extending proximally beyond the proximal retention structure 214. The barrier member 230 may include a mesh-like structure comprising a variety of woven or interlaced biocompatible polymers, metals and/or fabrics as are known in the art. The mesh-like structure may include an weaving pattern that provides openings or holes of sufficient size to allow bile and gallstones to pass through, while preventing or inhibiting the flow of digestive material (*e.g*., food etc.) entering the gallbladder and/or occluding the first lumen 212 of the elongate tubular member 210. It should be appreciated that the flexible composition and tapered design of the mesh-like structure may allow the barrier member 230 to deflect or bend in the direction of flow of digestive materials within the duodenum such that the opening 232 of the barrier member 230 is oriented to prevent or inhibit the entry of digestive materials. It should also be appreciated that the mesh-like structure is sufficiently stretchable to be received over the outer surface of a medical device (*e.g*., endoscope etc.) without permanently deforming.

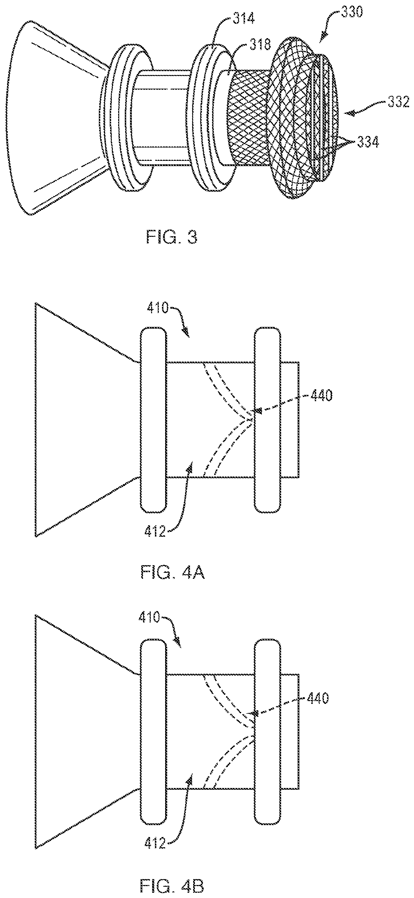

Referring to FIG. 3, the barrier member 330 may alternatively include a more rigid mesh-like cap disposed about a portion of the cylindrical saddle region 318 extending proximally beyond the proximal retention structure 314. As above, the barrier member 330 may be comprised of a variety of woven or interlaced biocompatible polymers, metals and/or fabrics as are known in the art. The opening 332 of the barrier member 330 may include a series of longitudinally-spaced bars 334 to prevent or inhibit the entry of digestive materials, as discussed above.

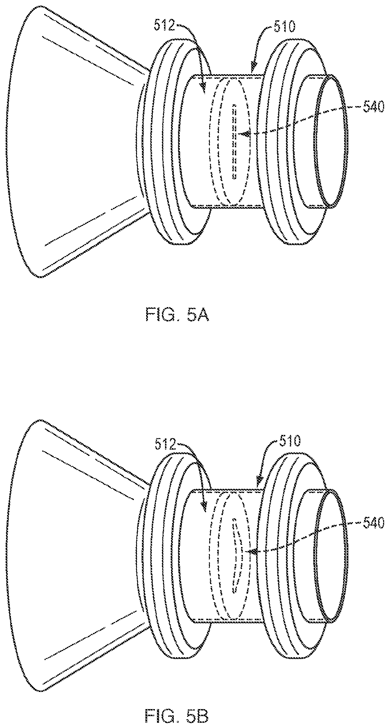

In addition (or as an alternative) to the barrier members, the first lumen 412 of the elongate tubular body 410 may further include a one-way valve 440 (*e.g*., duck-bill valve) movable between closed (FIG. 4A) and open (FIG. 4B) configurations to further prevent or inhibit the entry of digestive materials, without impeding the flow of bile and/or gallstones. In another embodiment, the first lumen 512 of the elongate tubular body 510 may further include a one-way slit valve 540 movable between closed (FIG. 5A) and open (FIG. 5B) configurations. Examples of such valves are described in U.S. Patent Publication No. 2012/0226243.

Such valves may comprise a variety of suitable biocompatible and non-degradable materials, including any of the polymers discussed herein.

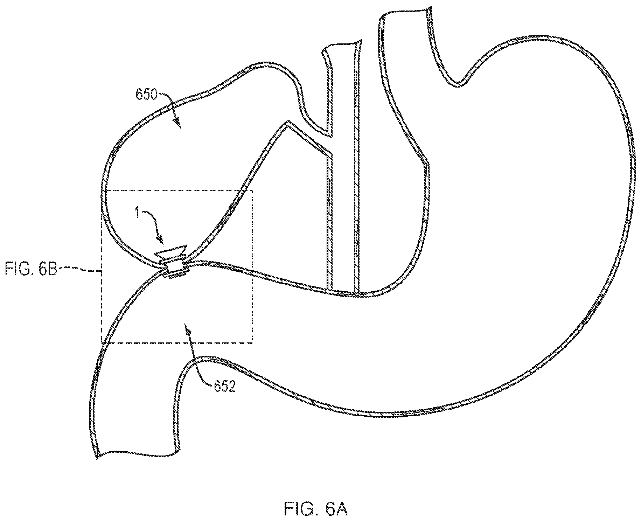

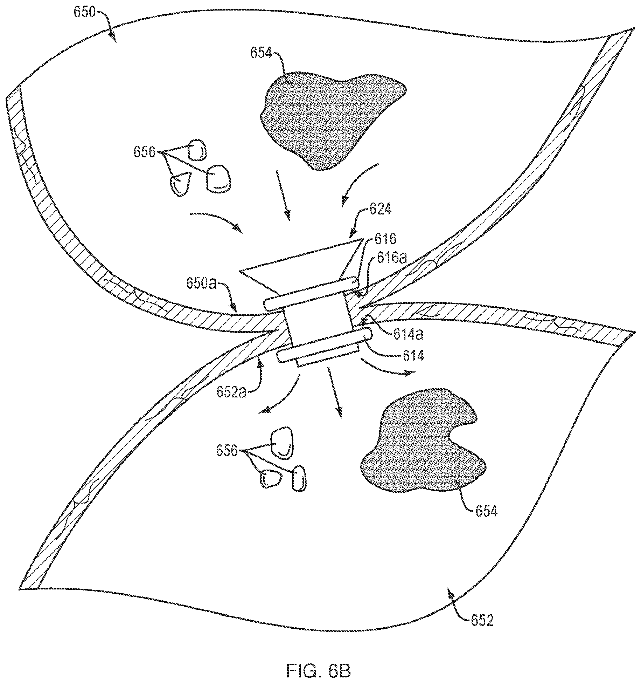

As illustrated in FIG. 6A, the drainage conduit 1 of the present disclosure may be positioned within a pre-formed anastomosis connecting the gallbladder 650 and duodenum 652. Referring to the expanded view of FIG. 6B, the drainage conduit 1 may be positioned within the anastomosis such that the planar surface 616a of the distal retention structure 616 is positioned against or adjacent to the inner wall surface 650a of the gallbladder 650, and the planar surface 614a of the proximal retention structure 614 is positioned against or adjacent to the inner wall surface 652a of the duodenum 652. It should be appreciated that direct contact between the proximal and distal retention structures 614, 616 and the respective tissue wall surfaces 650a, 652a of the gallbladder 650 and duodenum 652 is not necessary to prevent the drainage conduit 1 from moving into the either the gallbladder 650 or duodenum 652. Indeed, in one embodiment, such direct contact is preferably avoided to minimize tissue irritation resulting from prolonged contact with the proximal and distal retention structures 614, 616. As indicated by the direction of the arrows, the open central lumen 624 of the drainage conduit 1 may allow continual flow of gallstones 656 and bile 654 into the duodenum 652 for removal by the body's natural course.

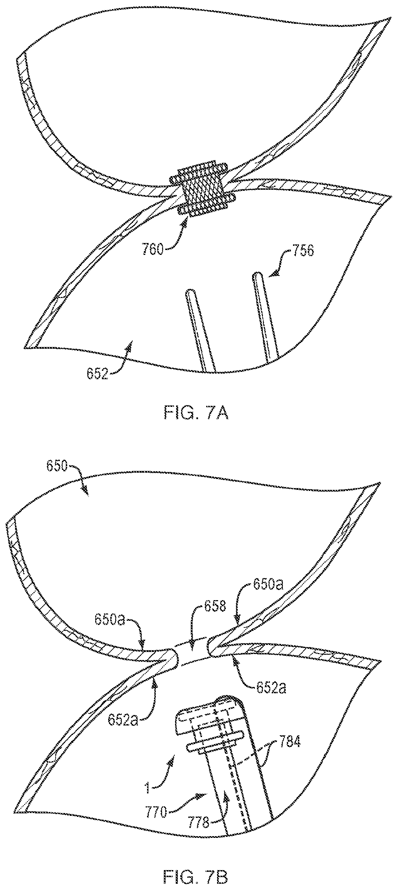

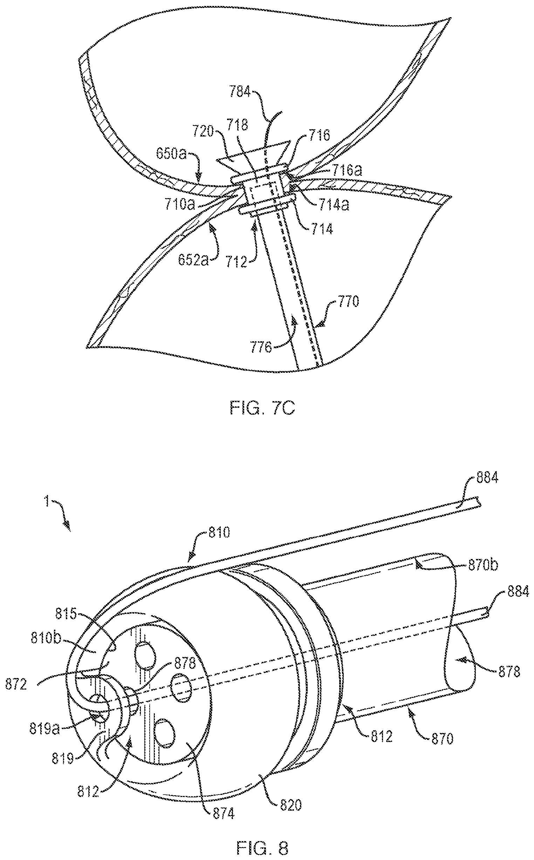

Referring to FIGS. 7A-7C, in practice and by way of example, a previously positioned tissue anchor 760 may be removed using a suitable device, such as grasping member 756, which is advanced through a working channel of an endoscope (not depicted) positioned within the duodenum 652 (FIG. 7A). After the tissue anchor has been removed, an endoscope 770 or other suitable delivery system may be used to advance a drainage conduit 1 in a first configuration to the anastomosis 658 (FIG. 7B). Referring to FIG. 7B, it should be appreciated that the tissue anchor depicted in FIG. 7A was implanted within the patient for a sufficient amount of time, typically 6-9 months, to allow the opposing tissue layers 650a, 652a of the gallbladder 650 and duodenum 652 to fuse and provide a permanent anastomosis 658. The fused tissue layers provide sufficient strength and flexibility to allow the drainage conduit 1 to be gently advanced into the anastomosis without tearing either of the tissue layers. As discussed in greater detail below, the drainage conduit 1 may be maintained in the first delivery configuration using a tether 784 in the form of a simple loop that extends the length of a working channel 778 of the endoscope 770 and back along the outer surface of the endoscope. As illustrated in FIG. 7C, the fused tissue layers 650a, 652a are sufficiently elastic to press and seal against the outer surface 710a of the cylindrical saddle region 718 between the proximal and distal retention structures 714, 716. The opposing planar surfaces 714a, 716a of the proximal and distal retention structures 714, 716 may contact the respective inner wall surfaces 650a, 652a to maintain the drainage conduit 1 within the anastomosis. Still referring to FIG. 7C, with the drainage conduit 1 positioned within the anastomosis, the funnel member 720 may be moved to the first deployed configuration by releasing a first end of the tether 784 and pulling the second end of the tether through a working channel 776 of the endoscope 770. The endoscope 770 may then be retracted proximally and removed from the first lumen 712 of the drainage conduit and withdrawn from the patient.

FIG. 8 provides an enlarged frontal view of the drainage conduit delivery system of FIG. 7B. In one embodiment, the distal end 874 of an endoscope 870 may be disposed within the first lumen 812 of the elongate tubular body 810. The distal end 815 of the elongate tubular body 810 may include a tab 819 that extends from the inner surface 810b into a portion of the first lumen 812. The tab 819 may serve as a stopping member which the distal end 874 of the endoscope 870 may push/press against as the endoscope 870 is advanced through the body passages (e.g., alimentary canal) towards the anastomosis. It should be appreciated that the tab 819 may be sufficiently small that it does not substantially block the flow of fluids and materials through the open central lumen. In addition, the tab 819 may be comprise a sufficiently deformable material such that it folds against the inner surface 810b of the first lumen 812 as fluids and materials flow through the open central lumen.

The tab 819 may further include an aperture 819a configured to receive a tether 884 in the form of a simple loop that extends through a working channel 878 and returns along an outer surface 870b of the endoscope 870. The funnel member 820 may be retained in the second configuration against a portion of the outer surface of the cylindrical saddle region by applying tension to both free ends of the tether 884, which extend beyond the proximal end of the endoscope (not shown). The proximal tension applied to the free ends of the tether 884 also ensures that the distal end 872 of the endoscope 870 remains in contact with the surface of the tab 819 as the delivery system is advanced through the body passages towards the anastomosis. In another embodiment, forceps may be passed through the working channel of the scope, and grasp a tab on the drainage conduit. The scope, drainage conduit, and forceps may all be then advanced in unison to position the drainage conduit into the anastomosis. Once in place, the forceps may be opened (released), thus releasing the drainage conduit. The forceps and scope may then be removed from the drainage conduit.

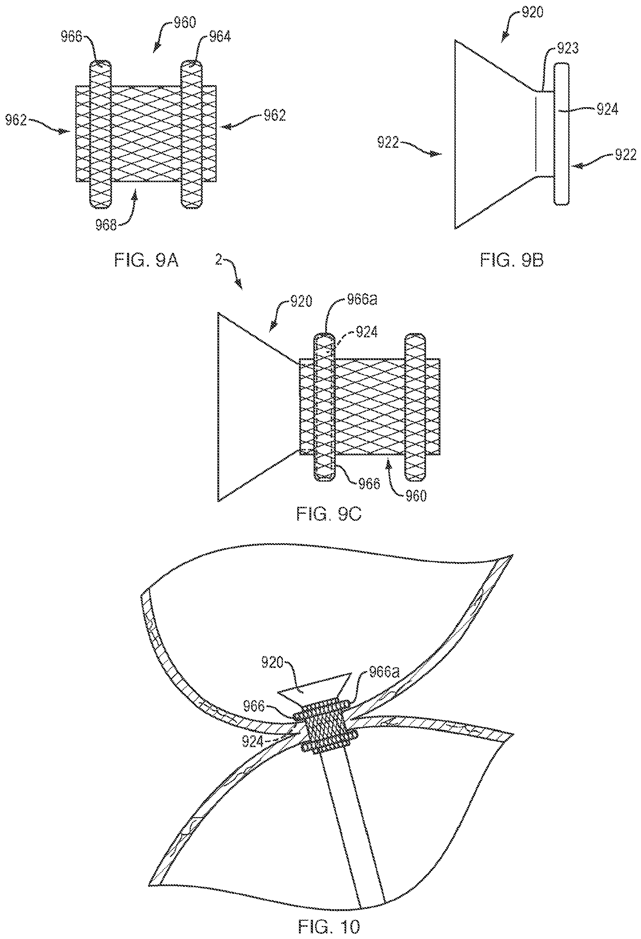

FIGS. 9A-9C illustrate an alternative embodiment of a drainage system 2, which may include a funnel member 920 configured to be attached to a tissue anchor 960. As illustrated in FIG. 9A, the tissue anchor 960 may include a cylindrical saddle region 968 defining a first lumen 962 with outward extending proximal and distal retention structures 964, 966. As illustrated in FIG. 9B, the funnel member 920 may define a second lumen 922 and include a proximal flange 924 extending outward from a proximal end 923. The funnel member 920 and proximal flange 924 may be comprise a variety of non-degradable and biocompatible polymeric materials, as discussed above. In one embodiment, the funnel member 920 and proximal flange 924 may be unitarily formed by polymeric extrusion process, as is known in the art. In addition, or alternatively, the proximal flange 924 may be permanently affixed (*i.e*., adhered, bonded, attached etc.) to an outer surface of the funnel member 920 using suitable glues, adhesives, resins or other polymer bonding techniques. As illustrated in FIG. 9C, the proximal flange 924 may be configured to form an interference fit with an inner surface 966a of the distal retention structure 966. The proximal flange 924 may be comprise a sufficiently flexible material that it deforms or collapses as the funnel member 920 is advanced through the first lumen 962 of the tissue anchor 960, and returns to its original shape to expand into and engage the inner surface 966a of the distal retention structure.

As illustrated in FIG. 10, in one embodiment, the funnel member 920 may be disposed in a compressed configuration within the lumen of an endoscope. The endoscope may have an outer diameter which is less than the inner diameter of the first lumen 962, such that the distal end of the endoscope may be positioned within the tissue anchor 960 just proximal to the distal retention structure 966. The funnel member 920 may be deployed from the distal end of the endoscope using *e.g.,* a pushrod slidably disposed within the endoscope lumen. As the funnel member 920 exits the endoscope lumen it may move from the compressed configuration to an expanded (*i.e.,* relaxed) configuration such that the funnel member expands within the gallbladder and the proximal flange engages the inner surface 966a of the distal retention structure 966.

## Claims

1. A drainage conduit (1; 2), comprising:
an elongate tubular body (110; 210; 410; 510; 810; 960) defining a first lumen (112; 212; 412; 512; 712; 812; 962), the elongate tubular body (110; 210; 410; 510; 810; 960) comprising:
a proximal retention structure (114; 214; 314; 614; 714; 964),
a distal retention structure (116; 616; 716; 966), and
a cylindrical saddle region (118; 218; 318; 718; 968) extending therebetween; and
a funnel member (120; 720; 920) attached to a distal end (115; 815) of the elongate tubular body (110; 210; 410; 510; 810; 960),
wherein the funnel member (120; 720; 920) is configured to move between a first, deployed configuration and a second, delivery configuration, and
wherein, when in the first, deployed configuration, the funnel member (120; 720; 920) and elongate tubular body (110; 210; 410; 510; 810; 960) define an open central lumen (624) to provide a flow path therethrough, said drainage conduit being **characterized in that** the funnel member (120; 720; 920) folds back along an outer surface of the elongate tubular body (110; 210; 410; 510; 810; 960) when in the second, delivery configuration.

2. The drainage conduit of claim 1, wherein the funnel member (120; 720; 920) comprises a compliant or semi-compliant material.

3. The drainage conduit of any of claims 1-2, wherein the proximal and distal retention structures (114, 116; 214; 314; 614, 616; 714, 716; 964, 966) extend outward from an outer surface (110a; 710a) of the elongate tubular body (110; 210; 410; 510; 810; 960).

4. The drainage conduit of any of claims 1-3, wherein the proximal and distal retention structures (114, 116; 214; 314; 614, 616; 714, 716; 964, 966) extend perpendicular to a circumference of the elongate tubular body (110; 210; 410; 510; 810; 960).

5. The drainage conduit of any of claims 1-4, wherein a diameter (114a, 116a) of the proximal and distal retention structures (114, 116; 214; 314; 614, 616; 714, 716; 964, 966) is larger than a diameter (118a) of the cylindrical saddle region (118; 218; 318; 718; 968).

6. The drainage conduit of any of claims 1-5, wherein the proximal and distal retention structures (114, 116; 214; 314; 614, 616; 714, 716; 964, 966) include opposing planar surfaces (114b, 116b; 614a, 614b; 714a, 716a) configured to contact a surface of opposing tissue walls (650a, 652a).

7. The drainage conduit of any of claims 1-6, wherein the proximal retention structure (114; 214; 314; 614; 964) is configured to contact a first tissue layer (652a), and the distal retention structure (116; 616; 966) is configured to contact a second tissue layer (650a).

8. The drainage conduit of any of claims 1-7, further comprising a barrier member (230; 330) disposed about a portion of the elongate tubular body (210) extending proximally beyond the proximal retention structure (214; 314).

9. The drainage conduit of claim 8, wherein the barrier member (330) includes a mesh-like structure.

10. The drainage conduit of any of claims 1-9, further including a valve (440; 540) disposed within the first lumen (412; 512; 712) of the elongate tubular body (410; 510).

11. The drainage conduit of claim 10, wherein the valve (540) is a one-way slit valve.

12. The drainage conduit of any of claims 1-11, further comprising a tab (819) extending from an inner surface (810b) of the elongate tubular body (810) into the first lumen (812).

13. The drainage conduit of claim 12, wherein the tab (819) includes an aperture (819a) therein.

14. The drainage conduit of any of claims 12-13, wherein the tab (819) is disposed at a distal end (815) of the elongate tubular body (810).

## Patentansprüche

1. Drainage-Kanal (1; 2), der aufweist:
einen länglichen Röhrenkörper (110; 210; 410; 510; 810; 960), der ein erstes Lumen (112; 212; 412; 512; 712; 812; 962) bildet, wobei der längliche Röhrenkörper (110; 210; 410; 510; 810; 960) aufweist:
eine proximale Retentionsstruktur (114; 214; 314; 614; 714; 964),
eine distale Retentionsstruktur (116; 616; 716; 966) und
einen zylindrischen Sattelbereich (118; 218; 318; 718; 968), der sich dazwischen erstreckt; und
ein Trichterteil (120; 720; 920), das an einem distalen Ende (115; 815) des länglichen Röhrenkörpers (110; 210; 410; 510; 810; 960) angebracht ist,
wobei das Trichterteil (120; 720; 920) so konfiguriert ist, dass es sich zwischen einer ersten gesetzten Konfiguration und einer zweiten Abgabekonfiguration bewegt, und
wobei in der ersten gesetzten Konfiguration das Trichterteil (120; 720; 920) und der längliche Röhrenkörper (110; 210; 410; 510; 810; 960) ein offenes Zentrallumen (624) bilden, um einen durchgehenden Strömungsweg bereitzustellen,
wobei der Drainage-Kanal **dadurch gekennzeichnet ist, dass** sich das Trichterteil (120; 720; 920) entlang einer Außenfläche des länglichen Röhrenkörpers (110; 210; 410; 510; 810; 960) in der zweiten Abgabekonfiguration zurückklappt.

2. Drainage-Kanal nach Anspruch 1, wobei das Trichterteil (120; 720; 920) ein nachgiebiges oder semi-nachgiebiges Material aufweist.

3. Drainage-Kanal nach Anspruch 1 oder 2, wobei sich die proximale und distale Retentionsstruktur (114, 116; 214; 314; 614, 616; 714, 716; 964, 966) von einer Außenfläche (110a; 710a) des länglichen Röhrenkörpers (110; 210; 410; 510; 810; 960) nach außen erstrecken.

4. Drainage-Kanal nach einem der Ansprüche 1 bis 3, wobei sich die proximale und distale Retentionsstruktur (114, 116; 214; 314; 614, 616; 714, 716; 964, 966) senkrecht zu einem Umfang des länglichen Röhrenkörpers (110; 210; 410; 510; 810; 960) erstrecken.

5. Drainage-Kanal nach einem der Ansprüche 1 bis 4, wobei ein Durchmesser (114a, 116a) der proximalen und distalen Retentionsstruktur (114, 116; 214; 314; 614, 616; 714, 716; 964, 966) größer als ein Durchmesser (118a) des zylindrischen Sattelbereichs (118; 218; 318; 718; 968) ist.

6. Drainage-Kanal nach einem der Ansprüche 1 bis 5, wobei die proximale und distale Retentionsstruktur (114, 116; 214; 314; 614, 616; 714, 716; 964, 966) gegenüberliegende ebene Oberflächen (114b, 116b; 614a, 614b; 714a, 716a) aufweisen, die so konfiguriert sind, dass sie eine Oberfläche gegenüberliegender Gewebewände (650a, 652a) kontaktieren.

7. Drainage-Kanal nach einem der Ansprüche 1 bis 6, wobei die proximale Retentionsstruktur (114; 214; 314; 614; 964) so konfiguriert ist, dass sie eine erste Gewebeschicht (652a) kontaktiert, und die distale Retentionsstruktur (116; 616; 966) so konfiguriert ist, dass sie eine zweite Gewebeschicht (650a) kontaktiert.

8. Drainage-Kanal nach einem der Ansprüche 1 bis 7, ferner mit einem Barrierenteil (230; 330), das um einen Abschnitt des länglichen Röhrenkörpers (210) angeordnet ist, der sich über die proximale Retentionsstruktur (214; 314) hinaus proximal erstreckt.

9. Drainage-Kanal nach Anspruch 8, wobei das Barrierenteil (330) eine maschenartige Struktur aufweist.

10. Drainage-Kanal nach einem der Ansprüche 1 bis 9, ferner mit einem Ventil (440; 540), das im ersten Lumen (412; 512; 712) des länglichen Röhrenkörpers (410; 510) angeordnet ist.

11. Drainage-Kanal nach Anspruch 10, wobei das Ventil (540) ein Einwegschlitzventil ist.

12. Drainage-Kanal nach einem der Ansprüche 1 bis 11, ferner mit einer Lasche (819), die sich von einer Innenfläche (810b) des länglichen Röhrenkörpers (810) in das erste Lumen (812) erstreckt.

13. Drainage-Kanal nach Anspruch 12, wobei die Lasche (819) eine Öffnung (819a) darin aufweist.

14. Drainage-Kanal nach Anspruch 12 oder 13, wobei die Lasche (819) an einem distalen Ende (815) des länglichen Röhrenkörpers (810) angeordnet ist.

## Revendications

1. Conduit de drainage (1 ; 2), comprenant :
un corps tubulaire allongé (110 ; 210 ; 410 ; 510 ; 810 ; 960) définissant une première lumière (112 ; 212 ; 412 ; 512 ; 712 ; 812 ; 962), le corps tubulaire allongé (110 ; 210 ; 410 ; 510 ; 810 ; 960) comprenant :
une structure de retenue proximale (114 ; 214 ; 314 ; 614 ; 714 ; 964),
une structure de retenue distale (116 ; 616 ; 716 ; 966), et
une région de selle cylindrique (118 ; 218 ; 318 ; 718 ; 968) s'étendant entre elles ; et
un élément formant entonnoir (120 ; 720 ; 920) fixé à une extrémité distale (115 ; 815) du corps tubulaire allongé (110 ; 210 ; 410 ; 510 ; 810 ; 960),
dans lequel l'élément formant entonnoir (120 ; 720 ; 920) est configuré pour se déplacer entre une première configuration déployée et une seconde configuration de mise en place, et
dans lequel, lorsqu'il est dans la première configuration déployée, l'élément formant entonnoir (120 ; 720 ; 920) et le corps tubulaire allongé (110 ; 210 ; 410 ; 510 ; 810 ; 960) définissent une lumière centrale ouverte (624) pour fournir un chemin d'écoulement à travers ceux-ci, ledit conduit de drainage étant **caractérisé en ce que** l'élément formant entonnoir (120 ; 720 ; 920) se replie le long d'une surface externe du corps tubulaire allongé (110 ; 210 ; 410 ; 510 ; 810 ; 960) lorsqu'il est dans la seconde configuration de mise en place.

2. Conduit de drainage selon la revendication 1, dans lequel l'élément formant entonnoir (120 ; 720 ; 920) comprend un matériau souple ou semi-souple.

3. Conduit de drainage selon l'une quelconque des revendications 1-2, dans lequel les structures de retenue proximale et distale (114, 116 ; 214 ; 314 ; 614, 616 ; 714, 716 ; 964, 966) s'étendent vers l'extérieur depuis une surface externe (110a ; 710a) du corps tubulaire allongé (110 ; 210 ; 410 ; 510 ; 810 ; 960).

4. Conduit de drainage selon l'une quelconque des revendications 1-3, dans lequel les structures de retenue proximale et distale (114, 116 ; 214 ; 314 ; 614, 616 ; 714, 716 ; 964, 966) s'étendent perpendiculairement à une circonférence du corps tubulaire allongé (110 ; 210 ; 410 ; 510 ; 810 ; 960).

5. Conduit de drainage selon l'une quelconque des revendications 1-4, dans lequel un diamètre (114a, 116a) des structures de retenue proximale et distale (114, 116 ; 214 ; 314 ; 614, 616 ; 714, 716 ; 964, 966) est plus grand qu'un diamètre (118a) de la région de selle cylindrique (118 ; 218 ; 318 ; 718 ; 968).

6. Conduit de drainage selon l'une quelconque des revendications 1-5, dans lequel les structures de retenue proximale et distale (114, 116 ; 214 ; 314 ; 614, 616 ; 714, 716 ; 964, 966) incluent des surfaces planes opposées (114b, 116b ; 614a, 614b ; 714a, 716a) configurées pour entrer en contact avec une surface de parois de tissu opposées (650a, 652a).

7. Conduit de drainage selon l'une quelconque des revendications 1-6, dans lequel la structure de retenue proximale (114 ; 214 ; 314 ; 614 ; 964) est configurée pour entrer en contact avec une première couche de tissu (652a), et la structure de retenue distale (116 ; 616 ; 966) est configurée pour entrer en contact avec une seconde couche de tissu (650a).

8. Conduit de drainage selon l'une quelconque des revendications 1-7, comprenant en outre un élément formant barrière (230 ; 330) disposé autour d'une partie du corps tubulaire allongé (210) s'étendant de manière proximale au-delà de la structure de retenue proximale (214 ; 314).

9. Conduit de drainage selon la revendication 8, dans lequel l'élément formant barrière (330) inclut une structure en forme de treillis.

10. Conduit de drainage selon l'une quelconque des revendications 1-9, incluant en outre une soupape (440 ; 540) disposée à l'intérieur de la première lumière (412 ; 512 ; 712) du corps tubulaire allongé (410 ; 510).

11. Conduit de drainage selon la revendication 10, dans lequel la soupape (540) est une soupape à fente unidirectionnelle.

12. Conduit de drainage selon l'une quelconque des revendications 1-11, comprenant en outre une patte (819) s'étendant depuis une surface interne (810b) du corps tubulaire allongé (810) dans la première lumière (812).

13. Conduit de drainage selon la revendication 12, dans lequel la patte (819) inclut une ouverture (819a) à l'intérieur.

14. Conduit de drainage selon l'une quelconque des revendications 12-13, dans lequel la patte (819) est disposée à une extrémité distale (815) du corps tubulaire allongé (810).
